# EUROPÄISCHE PATENTSCHRIFT

(11) **EP 0 546 330 B1**
(45) Veröffentlichungstag und Bekanntmachung des Hinweises auf die Patenterteilung: **28.02.1996**
(21) Anmeldenummer: 92119164.9
(22) Anmeldetag: 09.11.1992
(51) Int. Cl.: A61F 5/01

(54) **Orthesengelenk**
Motion brace
Orthèse pourvue d'une articulation

(30) Priorität: 11.11.1991 DE 4137056
(43) Veröffentlichungstag der Anmeldung: 16.06.1993
(73) Patentinhaber: BIEDERMANN MOTECH GmbH, D-78054 Villingen-Schwenningen (DE)
(72) Erfinder: Biedermann, Lutz, c/o Biedermann Motech GmbH, W-7730 VS-Schwenningen (DE)
(74) Vertreter: Prüfer, Lutz H.

(56) Entgegenhaltungen:
- EP-A- 0 454 186
- WO-A-92/15264
- GB-A- 2 215 394
- US-A- 4 928 676

## Beschreibung

Die Erfindung betrifft ein Orthesengelenk nach dem Oberbegriff des Patentanspruches 1.

Ein derartiges Orthesengelenk ist aus der US-PS 4.886.054 bekannt. Das Orthesengelenk wird insbesondere als Kniegelenk für Beinorthesen verwendet. Damit die Lagerplatte nicht am Knie scheuert, weist eine nach der Lehre aus der US-Patentschrift gebaute offenkundig vorbenutzte Orthese an der dem Knie zugewandten Seite der Lagerplatte eine mit der Lagerpatte fest verbundene Pelotte auf. Der Nachteil bei dieser Pelotte ist, daß sie sich wegen der durch die zwei Gelenke sich ergebenden Bewegungsbahn bei jeder Beugung bzw. Streckung in ihrer Lage verändert. Mit anderen Worten bewegt sich die Pelotte stets am Knie auf und ab bzw. hin und her.

Aufgabe der Erfindung ist es, das Orthesengelenk so auszubilden, daß die Pelotte sich beim Beugen bzw. Strecken des Orthesengelenkes nicht mehr auf und ab bzw. hin und her bewegt.

Diese Aufgabe wird durch das in Patentanspruch 1 gekennzeichnete Orthesengelenk gelöst.

Nach einer Weiterbildung der Erfindung sind die Anschlußteile und das Verbindungsglied miteinander ineingriff gebracht, und ihre Bewegungsfreiheit ist durch verstellbare Anschläge einstellbar, so daß eine Anpassung an die Belastbarkeit des Knies erfolgt. Die Anschläge sind elastisch ausgebildet. Dadurch wird der Tragkomfort wesentlich verbessert.

Weiterbildungen der Erfindungen sind in den Unteransprüchen gekennzeichnet.

Weitere Merkmale und Zweckmäßigkeiten der Erfindung ergeben sich aus der Beschreibung eines Ausführungsbeispieles anhand der Figuren. Von den Figuren zeigen:
- Fig. 1: einen Schnitt entlang der Linie A-A in Fig. 3, in gestreckter Endstellung des Orthesengelenkes;
- Fig. 2: dieselbe Gelenkdarstellung in gebeugter Endstellung;
- Fig. 3: eine Draufsicht auf das Orthesengelenk von vorn;
- Fig. 4: das in Fig. 2 gezeigte Orthesengelenk von der Rückseite her mit gestrichelt angedeuteter Pelotte; und
- Fig. 5: das in Fig. 1 gezeigte Orthesengelenk von der Rückseite her mit gestrichelt angedeuteter Pelotte.

Das Orthesengelenk weist eine erste Lagerplatte (1) auf. Mit dieser ist ein Oberschenkel-Anschlußteil (2) über eine sich senkrecht zur Ebene der Lagerplatte erstreckende Lagerbuchse (3) schwenkbar verbunden. Ferner ist ein Unterschenkel-Anschlußteil (4) vorgesehen, welches über eine sich senkrecht zur Ebene der Lagerplatte erstreckende zweite Lagerbuchse (5) mit der Lagerplatte (1) schwenkbar verbunden ist. Ein Verbindungsglied (6) ist zwischen den beiden Anschlußteilen vorgesehen. Dieses ist an seinem einen Ende über eine dritte Lagerbuchse (7) schwenkbar mit dem Unterschenkel-Anschlußteil (4) und an seinem anderen Ende über eine vierte Lagerbuchse (8) mit dem Oberschenkel-Anschlußteil (2) schwenkbar verbunden.

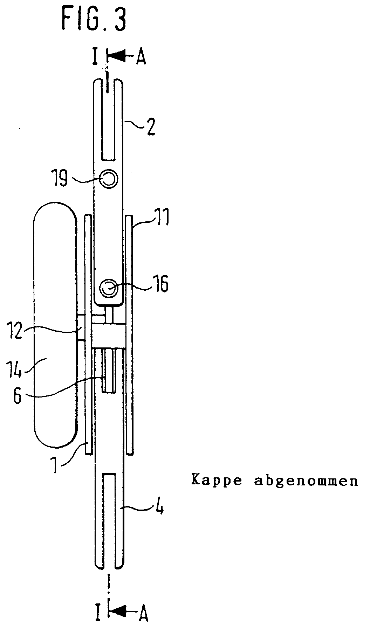

Die Verbindung zwischen dem Verbindungsglied und den Anschlußteilen ist so hergestellt, daß die Anschlußteile an ihren einander zugewandten Enden sich parallel zur Lagerplatte erstreckende schlitzförmige Ausnehmungen (9, 10) aufweisen. In diesen ist das Verbindungsglied bewegbar geführt. Wie am besten aus Fig. 3 ersichtlich ist, ist eine zweite Lagerplatte (11) vorgesehen, die insoweit zur ersten Lagerplatte spiegelsymmetrisch ausgebildet ist. Die jeweiligen Lagerbuchsen sind mit ihrem der ersten Lagerplatte abgewandten Ende in der zweiten Lagerplatte gelagert. Die Anschlußteile können sich zwischen den beiden Lagerplatten frei bewegen.

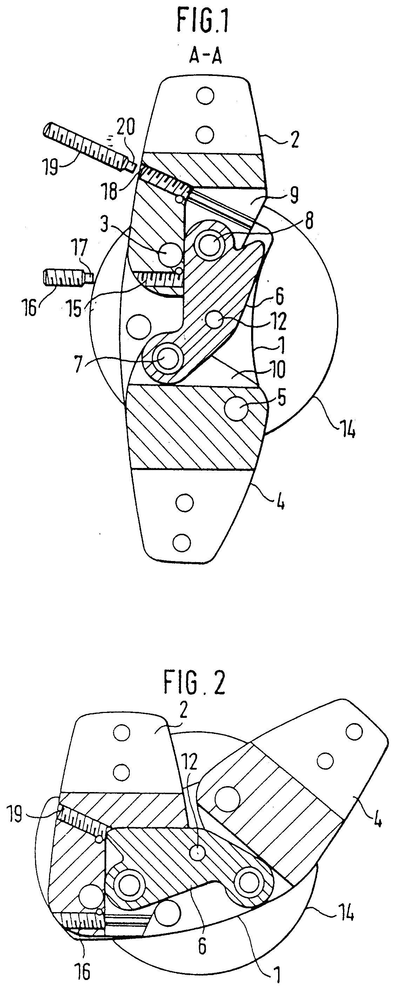

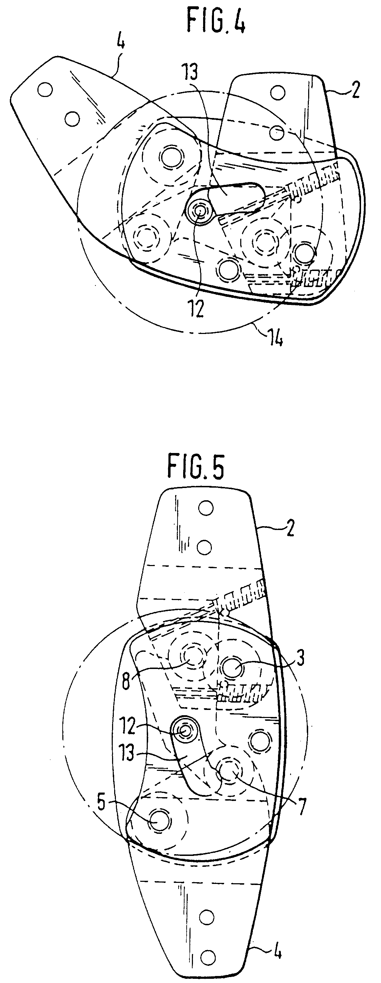

Die Lage der ersten bis vierten Lagerbuchsen ist in an sich bekannnter Weise so gewählt, daß die die erste und zweite Lagerbuchse verbindende erste Gerade und die die dritte und vierte Lagerbuchse verbindende zweite Gerade sich unter einem Winkel derart schneiden, daß sich bei der Bewegung der Anschlußteile bzw. der damit verbundenen Orthese aufgrund der zwei durch die erste und zweite Lagerbuchse gebildeten Gelenke eine Bewegung mit einem annähernd kreisförmigen Abschnitt und einem sich daran anschließenden annähernd schneckenförmigen Abschnitt ergibt. Das Verbindungsglied (6) weist eine Gewindebuchse (12) auf. In den Figuren 1 und 2 ist die Rückseite des die Gewindebuchse mit dem Verbindungsglied verbindenden Niets erkennbar, während die Gewindebuchse selbst in den Figuren 3 bis 5 zu sehen ist. Die erste Lagerplatte (1) weist eine Ausnehmung (13) auf, die so groß ist, daß die Gewindebuchse (12) sich bei der Bewegung des Orthesengelenkes frei durch die Ausnehmung hindurch bewegen kann. Eine Pelotte (14) ist mittels einer geeigneten Schraube über die Gewindebuchse (12) fest mit dem Verbindungsglied (6) verbunden. Die Gewindebuchse (12) ist, wie am besten aus Fig. 1 ersichtlich ist, auf einer Linie angeordnet, die sich senkrecht zur Längsachse des gestreckten Orthesengelenkes erstreckt und die einen etwa gleichen Abstand von der ersten Lagerbuchse (3) und von der zweiten Lagerbuchse (5) aufweist. Seitlich gesehen liegt die Stelle auf einer Geraden, die sich parallel zur Längsachse des gestreckten Orthesengelenkes erstreckt und etwa den gleichen Abstand von der ersten und von der zweiten Lagerbuchse aufweist. Durch die feste Verbindung der Pelotte mit dem Verbindungsglied (6) wird erreicht, daß diese sich bei der Bewegung des Orthesengelenkes nicht mehr auf und ab bzw. hin und her bewegt.

Wie am besten aus den Figuren 1 und 2 ersichtlich ist, ist in der Mittenebene des Oberschenkel-Anschlußteiles (2) eine erste sich parallel zur Lagerplatte erstreckende Gewindebohrung (15) vorgesehen. In dieser ist eine Gewindehülse (16), die einen durch eine innere Feder vorgespannten Anschlag (17) aufweist, eingeschraubt. Die Schraube ist in Abhängigkeit von den Bedürfnissen des Patienten so eingestellt, daß die in Fig. 1 gezeigte gestreckte Endstellung durch den Anschlag begrenzt wird. Ferner ist eine zweite Gewindebohrung (18) vorgesehen, in die eine entsprechende Gewindehülse (19) mit einem durch eine innnere Feder vorgespannten Anschlag (20) eingeschraubt ist. Durch die eingeschraubte Stellung der Gewindehülse mit dem Anschlag wird, wie insbesondere aus Fig. 2 ersichtlich ist, die gebeugte Endstellung des Orthesengelenkes begrenzt. Durch die Federvorspannung bzw. gewünschtenfalls auch eine durch einen Gummi erzeugte elastische Vorspannung wird das harte Anschlagen der Orthesenteile beim Tragen verhindert, so daß der Tragkomfort erheblich verbessert wird.

## Patentansprüche

1. Orthesengelenk mit einer ersten Lagerplatte (1), einem mit dieser über eine erste Lagerbuchse (3) schwenkbar verbundenen Oberschenkel-Anschlußteil (2),
einem mit der ersten Lagerplatte (1) über eine zweite Lagerbuchse (5) schwenkbar verbundenen Unterschenkel-Anschlußteil (4),
einem mit den beiden Anschlußteilen (2, 4) über eine dritte und eine vierte Lagerbuchse (7, 8) schwenkbar verbundenen Verbindungsglied (6),
dadurch gekennzeichnet, daß eine von dem Verbindungsglied (6) getragene Pelotte (14) vorgesehen ist.

2. Orthesengelenk nach Anspruch 1, dadurch gekennzeichnet, daß die Mitte der Pelotte (14) im wesentlichen auf einer Linie liegt, die senkrecht zur Längsrichtung des gestreckten Orthesengelenkes verläuft und annähernd den gleichen Abstand von der ersten und zweiten Lagerbuchse (3, 5) aufweist.

3. Orthesengelenk nach Anspruch 1 oder 2, dadurch gekennzeichnet, daß wenigstens eines der Anschlußteile in seinem dem anderen Anschlußteil zugewandten Bereich einen Schlitz (9, 10) aufweist, zwischen dem das Verbindungsglied (6) geführt ist.

4. Orthesengelenk nach einem der Ansprüche 1 bis 3, dadurch gekennzeichnet, daß auf der der ersten Lagerplatte (1) abgewandten Seite eine zweite Lagerplatte (11) vorgesehen ist
und eine der Lagerplatten eine Ausnehmung (13) aufweist, durch die sich die Verbindung zwischen dem Verbindungsglied (6) und der Pelotte (14) erstreckt.

5. Orthesengelenk nach einem der Ansprüche 1 bis 4, dadurch gekennzeichnet, daß eines der Anschlußteile (2) und das Verbindungsglied (6) über einen ersten und einen zweiten Anschlag (17, 20) in ihrer Relativbewegung einstellbar sind, wobei wenigstens einer der Anschläge einstellbar ist.

6. Orthesengelenk nach Anspruch 5, dadurch gekennzeichnet, daß wenigstens einer der Anschläge elastisch ausgebildet ist.

7. Orthesengelenk nach Anspruch 5 oder 6, dadurch gekennzeichnet, daß die Anschläge als in einer Gewindebohrung (15, 18) gehaltene Schraubhülsen (16, 19) mit federvorgespannten Anschlagköpfen (17, 20) ausgebildet sind.

8. Orthesengelenk nach einem der Ansprüche 5 bis 7, dadurch gekennzeichnet, daß die Anschläge (17, 20) in einem der Anschlußteile (2) vorgesehen sind und sich in den Schlitz (9) hinein erstrecken.

## Claims

1. A brace hinge having a first support plate (1), a femoral link member (2) pivotally connected to the first support plate through a first bearing bushing (3),
a tibial link member (2) pivotally connected to the first support plate (1) through a second bearing bushing (5),
a connecting part (6) pivotally connected to both the link members (2, 4) through third and fourth bearing bushings (7, 8),
characterized in that a pad (14) is provided being supported by the connecting part (6).

2. The brace hinge according to claim 1, characterized in that the center of the pad (14) is situated essentially on a line running perpendicularly to the longitudinal direction of the straightened brace hinge and having substantially the same distance from the first and second bearing bushings (3, 5).

3. The brace hinge according to claim 1 or 2, characterized in that at least one of the link members comprises a slit (9, 10) at the region thereof facing the other link member, the connecting part (6) being guided between the slit.

4. The brace hinge according to one of claims 1 to 3, characterized in that a second support plate (11) is provided on the side opposite to the first support plate, and
one of the support plates comprises a recess (13) through which the connection between the connecting part (6) and the pad (14) extends.

5. The brace hinge according to one of claims 1 to 4, characterized in that one of the link members (2) and the connecting part (6) is adjustable through first and second abutments (17, 20) in the relative movement, at least one of the abutments being adjustable.

6. The brace hinge according to claim 5, characterized in that at least one of the abutments is provided elastically.

7. The brace hinge according to claim 5 or 6, characterized in that the abutments are formed as screw sleeves (16, 19) supported in threaded bores (15, 18) and having spring biased abutments heads (17, 20).

8. The brace hinge according to one of claims 5 to 7, characterized in that the abutments (17, 20) are provided in one of the link members (2) and extend into the slit (9).

## Revendications

1. Jointure d'orthèse, à une première plaque d'appui, à un élément de raccord (2) pour raccordement à la cuisse, qui est relié, de façon pivotante, à ladite plaque via un premier coussinet (3),
à un élément de raccord (4) pour raccordement à la jambe, qui est relié, de façon pivotante, à ladite première plaque d'appui (1) via un deuxième coussinet (5),
à un élément d'assemblage (6), qui est relié, de façon pivotante, auxdits deux éléments de raccord (2, 4) via un troisième et un quatrième coussinet (7, 8) respectif,
**caractérisée en ce** qu'une pelotte (14) este prévue qui s'appuie sur ledit élément d'assemblage (6).

2. Jointure d'orthèse selon la revendication 1, **caractérisée en ce** que le centre de ladite pelotte (14) se trouve essentiellement sur une ligne qui s'étend orthogonalement sur la direction longitudinale de la jointure d'orthèse étendue, en étant environ équidistante dudit premier et deuxième coussinet (3, 5).

3. Jointure d'orthèse selon la revendication 1 ou 2, **caractérisée en ce** qu'au moins un desdits éléments de raccord est prévu, dans sa zone en face de l'autre élément de raccord, d'une fente (9, 10) à guider y entre ledit élément d'assemblage (6).

4. Jointure d'orthèse son la revendication 3, **caractérisée en ce** que du côte détourné de ladite première plaque d'appui, une deuxième plaque d'appui (11) est prévue,
et en ce qu'une desdites plaques d'appui présente un creux (13), à travers duquel s'étend le raccord entre ledit élément d'assemblage (6) est ladite pelotte (14).

5. Jointure d'orthèse selon une quelconque des revendications 1 à 4, **caractérisée en ce** qu'un desdits éléments de raccord (2) et ledit élément d'assemblage (6) sont ajustables moyennant une première et une deuxième butée (17, 20), à au moins une desdites butées étant ajustable.

6. Jointure d'orthèse selon la revendication 5, **caractérisée en ce** qu'au moins une desdites butées est formée à être élastique.

7. Jointure d'orthèse selon une des revendications 5 ou 6, **caractérisée en ce** que lesdites butées sont configurées sous forme d'une douille filetée (16, 19) à têtes de butée (17, 20) mises en précontrainte par des ressorts, qui est tenue dans un trou taraudé (15, 18).

8. Jointure d'orthèse selon une quelconque des revendications 5 à 7, **caractérisée en ce** que lesdites butées (17, 20) sont disposées dans un desdites éléments de raccord (2) et s'étendent à l'intérieur de ladite fente (9).
